# EUROPEAN PATENT SPECIFICATION

(11) **EP 4 061 125 B1**
(45) Date of publication and mention of the grant of the patent: **01.05.2024**
(21) Application number: 20807030.0
(22) Date of filing: 18.11.2020
(51) Int. Cl.: A01K 67/033

(54) **EGG LAYING DEVICE FOR FLIES**
EIERLEGEVORRICHTUNG FÜR FLIEGEN
DISPOSITIF DE PONTE POUR MOUCHES

(30) Priority: 19.11.2019 FR 1912881
(43) Date of publication of application: 28.09.2022
(73) Proprietor: La Compagnie des Insectes Industries, 77700 Bailly Romainvilliers (FR)
(72) Inventor: ROCHE-BRUYN, Anne-Sophie Marie Dominique Thibault, 75012 Paris (FR); ROBERT, Jean-Charles Camille Bernard, 86410 Dienne (FR)
(74) Representative: Cabinet Nony
(86) International application number: PCT/EP2020/082585
(87) International publication number: WO 2021/099417

(56) References cited:
- CN-U- 205 357 811
- FR-A1- 3 066 360
- US-A1- 2014 020 630

## Description

### Technical field

The invention relates to an egg laying device for flies, intended for entomoculture.

### Prior art

Entomoculture involves rearing insects, generally with the objective of producing food products for humans or animals.

Among insects, a distinction is made with respect to flies, the exploitation of which presents specific problems and requires particular solutions.

In particular, the applicant company is exploiting a method involving:
a) rearing flies and causing them to mate, then to lay eggs;
b) retrieving the eggs and ensuring that they hatch in order to obtain young larvae;
c) feeding the young larvae in order to make them grow, preferably by feeding them with food residues;
d) processing the adult larvae obtained in order to convert them into harvestable products and in particular into a protein-containing flour which is intended for animal feed and into an agricultural fertiliser.

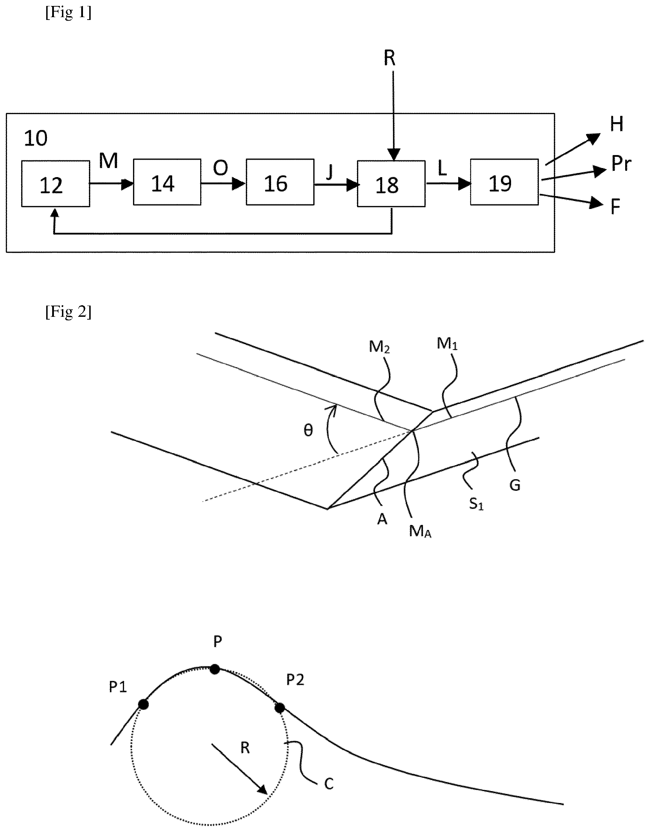

Carrying out such a method requires an installation 10 (see Figure 1) comprising a unit 12 for producing flies M, a unit 14 for producing eggs O which are laid by the flies, a unit 16 for producing young larvae J which are born from the eggs, a unit 18 for producing adult larvae L from the young larvae J and food residues R, and a unit 19 for processing the adult larvae L in order to produce an oil H, a protein-containing material Pr and a fertiliser F. Some of the larvae produced by the unit 18 are used by the unit 12 for producing flies.

The productivity of such an installation closely depends on the capacity thereof to ensure the optimum development conditions for the flies and the larvae. The equipment used is essential to this end.

Industrial exploitation also requires precise control of the different steps of the process. In particular, the flies are produced by batches of several thousands of individuals and it is desirable for the flies from the same batch to lay eggs substantially simultaneously. The larvae obtained then grow in the same manner, which improves the productivity.

FR 3 066 360 A1, US 2014/020630 A1 and CN 205 357 811 U disclose examples of egg lying devices for flies.

An object of the invention is to provide an egg laying device which allows the productivity of such an installation to be improved.

### Statement of invention

The invention is set out in the appended claims.

The invention sets out an egg laying device for flies, the device comprising:
- a fly cage preferably comprising a container comprising:
   - a base wall which defines a base surface which is orientated towards the interior of the cage, which is preferably horizontal, and
   - a lateral wall which surrounds the base surface and which defines a lateral surface which is orientated towards the interior of the cage, which is preferably vertical, and
- a laying nest which is fixed to the cage and
which is intended to receive the eggs laid by flies which are enclosed in the cage.

The base surface and/or the lateral surface has/have, over more than 90% of the area(s) thereof, a radius of curvature greater than 0.5 cm.

The base surface extends in a plane in an uninterrupted manner as far as the junction zone with the lateral surface, and does not have any raised portions or depressions, the height of which measured perpendicularly to the general plane of the base surface being greater than 10 mm.

The container defines a junction zone between the lateral surface and the base surface, the junction zone having at any point a radius of curvature greater than 1 cm.

The lateral wall comprises a plurality of planar faces, a junction zone between two adjacent faces having at any point a radius of curvature greater than 1 cm.

The term "internal surface" of the container is intended to refer to the surface of the walls thereof which delimit the internal volume thereof.

### Definitions

The "service position" is the position in which the egg laying device is used to cause flies enclosed in the cage to lay eggs in the laying nest.

The adjectives "lower" and "upper", "internal" and "external", "horizontal" and "vertical" make reference to orientations or positions in the service position.

The terms "horizontal" and "vertical" are intended to be understood to be an orientation forming with a completely horizontal and vertical plane, respectively, an angle less than 20°, preferably less than 15°, preferably less than 10°, preferably less than 5°, or less than 2°, or less than 1°. The "edges" of a surface are constituted by the points of this surface to which a line having a greater gradient has a gradient variation, also referred to as a "change of slope", greater than 45°. By extension, it is also said that the angle of the edge is greater than 45° or that the surface has a change of slope greater than 45°. An edge is a re-entrant edge when it defines an indentation. For example, Figure 2 illustrates a surface S₁ on which the line having the greatest slope G has a change of slope of θ at any point M_{A} of the line A. If the angle θ is greater than 45° for all the points of the line A, the line A is an edge. In practice, it is possible to measure the change of slope between two points M₁ and M₂ of the line having the greatest slope which are separated by a spacing of 3 mm, the spacing of 3 mm being measured by following the line.

The "smallest radius of curvature" at a point of a surface is the smallest radius of curvature considering all the directions. For example, at a point of a cylinder having a circular cross section, the smallest radius of curvature at a point of the cylindrical surface is the radius of the cylinder.

A surface has an area As and is constituted by a set of points. Each of these points has a smaller radius of curvature, which is identical to or different from that of the other points. The set of points of this surface, the smallest radius of curvature of which is greater than x cm (or less than x cm), covers an area Ax. The ratio *Ax*/*As* represents the percentage of the area of the surface which is covered by these points.

The surface "has a radius of curvature greater than x cm (or less than x cm) over more than y% of its area" when the ratio *Ax*/*As* is greater than y%, that is to say, when the set of points of this surface for which the smallest radius of curvature is greater than x cm (or less than x cm, respectively) covers more than y% of the area of this surface.

In practice, as illustrated in Figure 2, in order to measure a radius of curvature at a point P in a direction, there is marked, on the surface, a line which passes through the point P and which follows this direction. Subsequently, there are placed on this line two points P1 and P2 which are each spaced apart by 1.5 mm from the point P and which are arranged at each side of the point P. Then, there is marked a circular arc C which passes through the three points P1, P and P2. The radius of curvature R is the radius of this circular arc.

The term "equivalent diameter" of an opening or a surface is intended to be understood to mean the diameter of a disc which has the same area as this opening or as this surface, respectively.

A "junction zone" is an intermediate surface which connects two other surfaces and which, between these two other surfaces, is strictly concave, when viewed from the interior of the cage. A junction zone may particularly connect two planar surfaces which are orientated differently.

The term "blue" is intended to be understood to be a colour, the wavelength of which is between 472 nm and 490 nm.

The term "green" is intended to be understood to be a colour, the wavelength of which is between 490 nm and 573 nm.

The term "yellow" is intended to be understood to be a colour, the wavelength of which is between 573 nm and 584 nm, preferably between 575 nm and 579 nm.

Unless otherwise indicated, the term "to fix" means "to fix in a rigid manner". A "fixing" may be temporary, that is to say, it may allow release, or it may be definitive.

The terms "comprise", "define", "have" or "include" must be interpreted in a broad, non-limiting manner.

### Brief description of the Figures

Other features and advantages of the invention will be further appreciated from a reading of the following detailed non-limiting description and the examination of the appended drawings, in which:
- [Fig 1] Figure 1 schematically illustrates an entomoculture installation using flies;
- [Fig 2] Figure 2 schematically illustrates the definition of a re-entrant edge and a radius of curvature;
- [Fig 3] Figure 3 is a schematic perspective view of an example of an egg laying device according to the invention, the cover being detached;

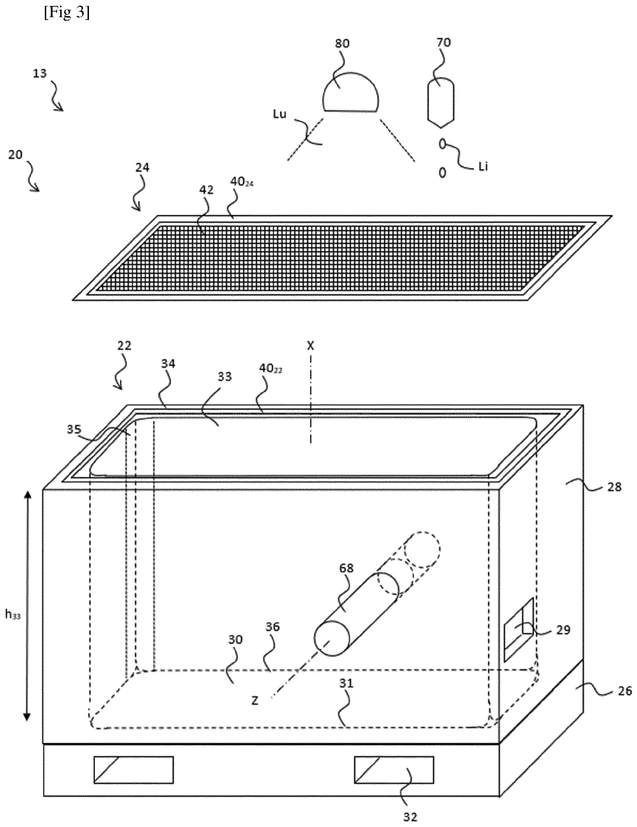

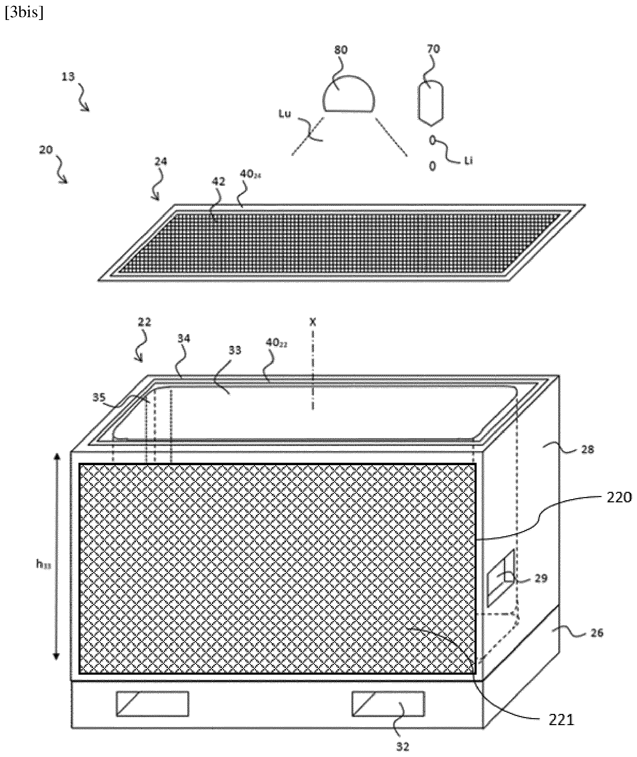

- [Fig 3bis] Figure 3bis is a view similar to figure 3 of an embodiment;

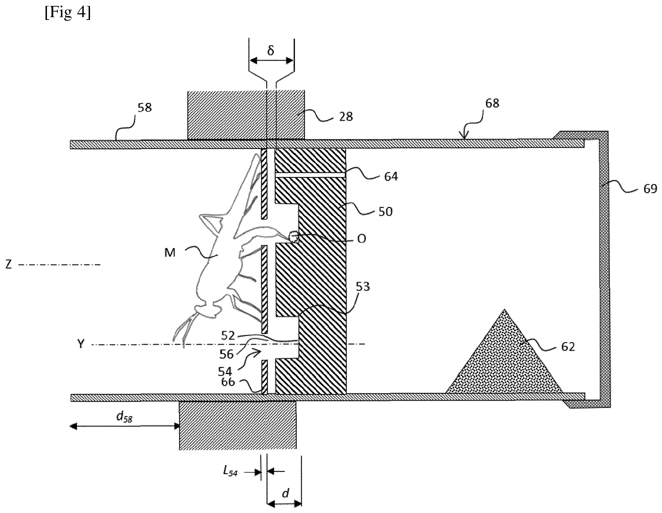

- [Fig 4] Figure 4 is a schematic longitudinal section of a simplified example of a detail of the device of Figure 3, the number of egg laying apertures preferably being much higher;

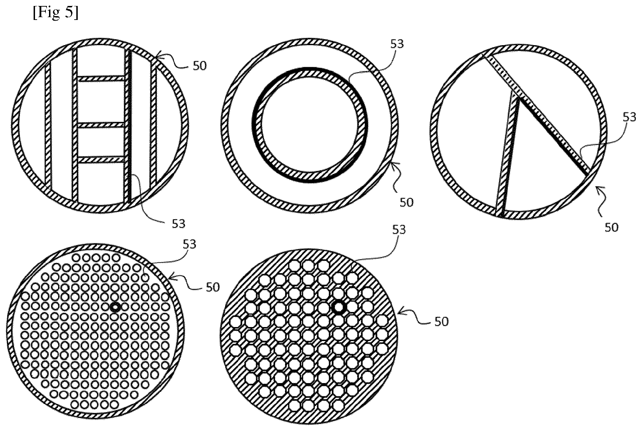

- [Fig 5] Figure 5 is a schematic front view of the examples of laying nests.

In the different Figures, identical or similar reference numerals are used to denote components or component portions which are identical or similar.

### Detailed description

Figure 3 and 3bis illustrate an example of an egg laying device 13 according to the invention. This device is intended for the exploitation of BSF flies, but the invention is not limited to this fly.

This device comprises a fly cage 20 and a laying nest 50.

### Fly cage

The fly cage 20 is intended to enclose a batch of flies M, which is preferably constituted by more than 500 flies, preferably more than 1000 flies, and/or less than 50,000 flies, preferably less than 20,000 flies.

The cage is preferably constituted by a container 22 and a cover 24 for closing the container 22.

The container 22, which is preferably monobloc, is preferably made from a polymer material, for example, polyethylene. It comprises a base wall 26 and a lateral wall 28 which together define the internal surface of the container. Preferably, the internal surface of the container does not have any undercut surface, that is to say, a surface which would prevent or inhibit the container from being removed from the mould along the axis X.

The base wall 26 internally defines a base surface 30, which is substantially planar and which, in the service position, preferably extends horizontally.

The lateral wall 28 defines, at the side of the interior of the cage, a lateral surface 33 which extends, preferably substantially perpendicularly to the base surface 30 as far as an upper rim 34. The base and lateral walls also define a junction zone 36 between the lateral surface 33 and the base surface 30. The junction zone 36 ensures a progressive transition between the lateral surface 33 and the base surface 30.

The internal surface of the container is thus constituted by the base surface 30, the lateral surface 28 and the junction zone 36 between these base and lateral surfaces.

The cage 20, preferably the container 22, more preferably the lateral wall 28, is preferably passed through by a connection aperture 29 which can selectively be closed and which is intended for introducing the flies into the cage. The connection aperture 29 preferably has a passage area greater than 50 cm², preferably greater than 100 cm², and/or preferably less than 1500 cm², preferably less than 1000 cm².

The base surface 30 which is delimited by an external contour 31 is continuous, that is to say that it remains planar and uninterrupted as far as its external contour, where it joins the junction zone 36 with the lateral surface 33. In particular, it does not comprise any through-opening, for example, a bung-hole which is intended for the discharge of liquid contained in the container 22. The term "planar base surface" is intended to be understood to mean that the base surface 30 does not have any raised portions or depressions, the height of which measured perpendicularly to the general plane of the base surface is greater than 10.

More preferably, the base surface 30 has a white, green, blue or yellow colour, preferably yellow, or is transparent. The colour spectrum is preferably between 472 nm and 600 nm.

The colour is preferably uniform. Surprisingly, the inventors have discovered that these colours were favourable for the reproduction of the flies.

In a preferred embodiment, the base wall 26 defines sleeves 32 which are suitable for introducing forks of a pallet truck. A plurality of egg laying devices according to the invention may thus be stacked in order to constitute a vertical farm according to the invention.

The height h₃₃ of the internal surface of the container is preferably constant. It is preferably greater than 0.4 m, preferably greater than 0.5 m and/or preferably less than 2 m, preferably less than 1.8 m or less than 1.5 m or 0.8 m. A height between 0.4 m and 1.8 m is particularly advantageous. This is because the inventors have discovered that such a height does not impede the flight of the flies and therefore promotes their well-being. When a pair of flies falls onto the base surface 30 in order to mate, however, the impact suffered by these flies remains insignificant and does not lead to them becoming separated.

The lateral wall 28 is preferably constituted by a polymer material, preferably polyethylene, preferably constituted from the same material as the base wall.

Preferably, the lateral surface 33 has a white, green, blue or yellow colour, preferably yellow, or is transparent. The colour is preferably uniform, preferably identical to the colour of the base surface. The colour spectrum is preferably between 472 nm and 600 nm.

Preferably, the junction zone 36 between the lateral surface 33 and the base surface 30 has a white, green, blue or yellow colour, preferably yellow, or is transparent. The colour is preferably uniform, preferably identical to the colour of the base surface. The colour spectrum is preferably between 472 nm and 600 nm.

The shape of the lateral wall 28 is not limiting. Preferably, however, the lateral wall 28 is constituted by a plurality of substantially planar faces, preferably more than 3 faces and/or less than 10 faces, preferably less than six faces, preferably four faces. The angle which is formed between two adjacent faces is preferably constant, whatever pair of two faces is being considered. In one embodiment, the faces of the lateral wall 28 all have the same length.

In the preferred embodiment, the lateral wall 28 has a plurality of planar faces which define a profile-member having a cross -section (that is to say, in a plane perpendicular to the axis X) which is polygonal, preferably rectangular, preferably square. The spatial requirement of the cage is thereby advantageously reduced.

In a particularly advantageous embodiment, the junction zone 35 between two adjacent faces which is delimited by broken lines in Figure 3, and which is preferably substantially vertical, does not define a re-entrant edge. In other words, the change in direction of the lateral surface 28 is progressive between these two faces. The junction zone 35 ensures a progressive transition between the two adjacent faces.

More preferably, the junction zone 36 between the lateral surface 33 and the base surface 30 does not have any re-entrant edge.

The absence of edges advantageously limits the clutches of eggs in the junction zones between the faces or between the lateral wall and the base wall.

In general, the internal surface of the container 22 defines only a minimum of re-entrant edges. On average, on the base surface 30 and/or on the lateral surface 33, or on the whole of the internal surface of the container, the cumulative length of the re-entrant edges is preferably such that, if *La* denotes the cumulative length of the re-entrant edges on the surface being considered, and S denotes the area S of this surface, *La*/*S* < 1200 mm/m², preferably *La*/*S* < 1000 mm/m², preferably *La*/*S* < 900 mm/m², preferably *La*/*S* < 850 mm/m², preferably *La*/*S* < 800 mm/m², without counting any re-entrant edges in the junction zone between the base surface and the lateral surface, preferably counting any re-entrant edges in the junction zone between the base surface and the lateral surface.

The orientation of the internal surface changes progressively everywhere (when it changes). The lateral surface 28 and the base surface 30 have a radius of curvature greater than 1 cm, preferably greater than 3 cm, preferably greater than 5 cm, preferably greater than 7 cm, and/or less than 30 cm.

The cover 24 is intended to close the container 22. It is preferably removable. More preferably, it is fixed to the upper rim 34 of the lateral wall 28. It can particularly be fixed to the container by means of a hook-and-loop strip of the Velcro^{®} type, the first and second portions 40₂₂ and 40₂₄ of which are bonded to the upper rim 34 and the periphery of the cover 24, respectively. The attachment and detachment of the cover 24 are thus very rapid. Furthermore, a continuous hook-and-loop strip effectively prevents the flies from moving towards the exterior of the cage.

The cover 24 is preferably open-worked so as to allow the external light, the ambient air and a liquid, preferably water, to pass. In the preferred embodiment, the cover comprises a grill 42, the mesh of which is sized to prevent the passage of a fly. Preferably, each mesh has an equivalent diameter less than 2 mm, preferably less than 1.5 mm. A flexible grill of the type used to constitute anti-mosquito nets is highly suitable.

In the embodiment shown in Figure 3bis, container 22 of fly cage 20 may have at least one through-opening 220 on the lateral wall 28. This through-opening 220 is rectangular.

The through-opening 220 shall extend over more than 90% of the face of the lateral surface carrying it.

The through-opening 220 extends over more than 90% of the area of the face of the lateral surface carrying it.

The through-opening has a mesh 221 identical to the mesh 221 on cover 24.

### Laving nest and egg laying aperture

The laying nest 50 is intended to receive and fix eggs laid by the flies which are enclosed in the cage.

The laying nest 50 is fixed to the cage, preferably in a removable manner. It defines an egg laying zone 52 which preferably defines at least one re-entrant edge 53, for example, in the form of an angle or a corner, as illustrated in Figure 4. Preferably, the angle of the re-entrant edge 53 is greater than 60°, 80° and/or less than 120°, 100°, preferably of approximately 90°. Preferably, at least one re-entrant edge 53 of the egg laying zone 52 has a length greater than 5 mm, preferably greater than 10 mm, preferably greater than 20 mm, more preferably greater than 40 mm, 60 mm or 100 mm. In a preferred embodiment, the re-entrant edge 53 is closed on itself, that is to say that it forms a closed loop. The inventors have discovered that such an edge is particularly effective for bringing about the laying of eggs.

Figure 5 illustrates different examples of laying nests 50 which have different re-entrant edges 53. In each example, a re-entrant edge has been drawn with bold lines. The length of this edge is the length of the bold line.

The laying nest 50 is preferably constituted by a polymer material.

As illustrated in Figure 4, the laying nest 50 is preferably at the outer side of the cage and is arranged near an egg laying aperture 54 which is arranged through the cage 20, preferably through the container 22 and more preferably through the lateral wall 28.

The egg laying aperture 54 preferably has a tubular shape. In cross section, that is to say, in a plane of section perpendicular to the axis Y of the egg laying aperture, the egg laying aperture 54 has a contour without any angle, which is preferably strictly concave, preferably circular or oval. The risk of eggs being laid in an undesirable manner in the egg laying aperture 54 itself is thus advantageously limited.

In a cross section, preferably in any cross section, the equivalent diameter of the egg laying aperture 54 is preferably greater than 1 mm, preferably greater than 2 mm, preferably greater than 2.5 mm and/or preferably less than 5 mm, preferably less than 4 mm.

The length L₅₄ of the egg laying aperture 54, measured along the axis Y, is preferably greater than 0.1 mm, preferably greater than 0.3 mm, preferably greater than 0.5 mm, preferably greater than 1 mm, and/or preferably less than 3 mm, preferably less than 2.5 mm, preferably less than 2 mm.

The inventors have discovered that such dimensions of the egg laying aperture 54 limit the risk of eggs being laid in an undesirable manner in the egg laying aperture while preventing the passage of the flies.

The egg laying aperture 54 opens, towards the exterior of the cage, via an external opening 56. The egg laying zone 52 which is associated with the egg laying aperture 54 is the zone of the laying nest on which the flies can lay eggs when they are laying eggs through the egg laying aperture 54. It is preferably ribbed.

The laying nest 50 is arranged in relation to the external opening 56 in such a manner that the egg laying zone 52 is facing the external opening 56 and is preferably spaced apart from the external opening 56. Advantageously, eggs can thus be deposited on the egg laying zone 52 without coming into contact with the edge of the external opening 56, and more generally without coming into contact with the egg laying aperture 54.

Preferably, the egg laying zone 52 is spaced apart from the external opening 56 by a spacing *d* greater than 3 mm, preferably greater than 5 mm, preferably greater than 8 mm, and/or preferably less than 20 mm, preferably less than 15 mm, preferably less than 10 mm.

Preferably, the egg laying zone 52 is spaced apart from the internal opening of the egg laying aperture 54, which is opposite the external opening and via which the egg laying aperture opens in the cage, by a spacing (*d*+L₅₄) greater than 4.5 mm, preferably greater than 6.5 mm, preferably greater than 9.5 mm and/or preferably less than 21.5 mm, preferably less than 16.5 mm, preferably less than 11.5 mm.

More preferably, the laying nest 50 is not in contact with the portion of the lateral wall which defines the egg laying aperture. Preferably, it is spaced apart therefrom by a spacing δ greater than 0.1 mm and/or less than 5 mm.

Preferably, the egg laying aperture 54 is provided in a region of the cage which, in the service position, receives less light than the remainder of the internal surface of the container 22, and in particular receives less light than the remainder of the lateral surface. To this end, particularly when the interior of the cage 20 receives light through the cover 24, the cage comprises an obstacle 58 which prevents the light from directly illuminating the egg laying aperture 54. This obstacle 58 may be constituted by, for example, a cap or a sleeve which opens at one side inside the cage and, at the opposite side, towards the egg laying aperture 54. The shape of the sleeve is not limited and may, for example, be tubular. The sleeve may or may not be rectilinear and may particularly have a baffle. The inventors have found that a protection of the egg laying aperture 54 in order to prevent it from being directly illuminated effectively promotes the egg laying through the egg laying aperture.

### Odoriferous source

In a preferred embodiment, the egg laying device further comprises an odoriferous source 62 which generates an odour which attracts the flies, preferably a putrefaction odour.

The form of the odoriferous source 62 is not limiting. Preferably, the odoriferous source 62 is dry. It may in particular be in the form of a solid block or a powder. In an embodiment, the odoriferous source 62 is humid, for example, constituted by a wick soaked in an odoriferous liquid.

The odoriferous source 62 is arranged so as to attract the flies present in the cage towards the egg laying aperture 54. Preferably, the odoriferous source 62 is arranged at the side of the external opening 56 of the egg laying aperture 54 in such a manner that, for the flies, the odour comes from the egg laying aperture 54.

In a particularly preferred embodiment, the odoriferous source 62 is arranged at the side of the laying nest 50 opposite the egg laying zone 52. More preferably, the laying nest 50 is perforated with one or preferably several channels 64 which allow(s) the passage of the odours emitted by the odoriferous source 62 towards the egg laying aperture 54. Preferably, any channel 64 is formed to prevent the passage of the eggs of the flies. The equivalent diameter of the cross section of the channel 64 is preferably less than 3 mm, preferably less than 2 mm and/or preferably greater than 0.5 mm.

At least one channel 64 is preferably arranged near the egg laying aperture 54, preferably at less than 30 mm, preferably at less than 20 mm, preferably at less than 10 mm from the egg laying aperture 54. Preferably, at least one channel 64 is arranged at less than 20 mm, preferably at less than 10 mm from any egg laying aperture 54.

The cage 20 preferably has a plurality of egg laying apertures, preferably more than 10, preferably more than 50, preferably more than 100 egg laying apertures 54. The risk of obstructing an egg laying aperture 54 is thus limited. Preferably, the cage has less than 10,000, preferably less than 5000, preferably less than 1000 egg laying apertures.

The spacing between two adjacent egg laying apertures 54 is preferably greater than 1 mm, preferably greater than 2 mm, and/or preferably less than 10 mm. The inventors have discovered that such a distribution of the egg laying apertures offers a good compromise between dimensions and efficiency.

Preferably, the egg laying aperture(s) is/are arranged in an egg laying plate 66 which is preferably removable, that is to say, detachable. The egg laying plate 66, which is preferably planar, may particularly be in the form of a grill perforated by egg laying apertures which are substantially circular.

More preferably, the egg laying plate 66 extends parallel with the laying nest 50, preferably without being in contact with it. Preferably, the smallest spacing δ between the egg laying plate 66 and the laying nest, when measured along the axis Y of the egg laying apertures 54, is greater than 0.1 mm and/or less than 5 mm.

The egg laying plate 66 is preferably sufficiently rigid not to become deformed. Advantageously, the spacing between the external openings of the egg laying apertures 54 and the corresponding egg laying zones 52 is thus constant over time, and preferably identical whatever the egg laying aperture being considered. The distribution of the eggs between the different egg laying zones is advantageously more uniform. Preferably, this spacing *d* is greater than 3 mm, preferably greater than 5 mm, preferably greater than 8 mm and/or preferably less than 20 mm, preferably less than 15 mm, preferably less than 10 mm, whatever the egg laying aperture being considered.

The thickness of the egg laying plate 66 defines the length L₅₄ of the egg laying apertures. It is preferably constant, preferably greater than 0.1 mm, preferably greater than 0.3 mm, preferably greater than 0.5 mm, preferably greater than 1 mm, and/or preferably less than 3 mm, preferably less than 2.5 mm, preferably less than 2 mm.

Preferably, the spacing (*d*+L₅₄) is greater than 4.5 mm, preferably greater than 6.5 mm, preferably greater than 9.5 mm, and/or preferably less than 21.5 mm, preferably less than 16.5 mm, preferably less than 11.5 mm.

The egg laying plate 66 is preferably made from a plastics material or a metal, preferably from a stainless steel.

An egg laying zone of the laying nest is associated with each egg laying aperture. In a preferred embodiment, all the egg laying zones 52 are defined by the same monobloc laying nest. The laying nest 50 may in particular extend parallel with the egg laying plate 66. When observed perpendicularly to the egg laying plate, it may have an external contour which is substantially identical to the contour of the egg laying plate 66 so as to extend in alignment with the egg laying plate 66 in the service position, as illustrated in Figure 5.

The laying nest 50 may extend vertically, as illustrated in Figure 4. However, the vertical orientation is not limiting. In particular, in one embodiment which is not illustrated, the laying nest 50 may extend horizontally.

The laying nest 50 is preferably fixed to the cage. Advantageously, the egg laying device is more easily manipulable, in particular in order to constitute a vertical farm. The laying nest 50 may be fixed to the cage 20 by any means.

In a preferred embodiment, the laying nest 50 and the egg laying plate 66 are arranged in a tubular support 68, for example, a tube, for example, of PVC, which has an internal diameter greater than 100 mm and which extends through the container 22, preferably the lateral wall 28. They preferably extend substantially perpendicularly to the axis Z of the tubular support 68 and more preferably have an external contour which substantially complements the internal surface of the tubular support 68 so as to substantially close the tubular support 68.

The tubular support 68 preferably protrudes inside the cage 20, preferably over a length *d₅₈* which is greater than 5 cm, preferably greater than 10 cm and/or less than 30 cm, preferably less than 20 cm. Thus, it may constitute an obstacle 58 to the light which irradiates the interior of the container, without increasing the spatial requirement of the device.

The device preferably comprises a stopper 69 for closing the tubular support at the side opposite the cage.

### Liquid source

The egg laying device 13 preferably comprises a liquid source 70, preferably a water source, which is arranged so as to be able to introduce the liquid Li inside the cage, preferably as a result of gravitational force, preferably through the cover.

The form of the liquid source is not limiting.

### Light source

An egg laying device according to the invention also preferably comprises a light source 80, which is preferably external with respect to the cage and which is suitable for exposing the interior of the cage to a light Lu and which is preferably suitable for not directly illuminating the egg laying aperture(s) 54.

The form of the light source is not limiting.

### Operation

The operation of the egg laying device 13 according to the invention follows directly from the above description. It is described in the preferred, non-limiting embodiment of the device.

Initially, the cage 20 which does not contain any flies, but also preferably no other object either, is closed by attaching the flexible cover 24 over the entire length of the upper rim 34 of the lateral wall 28. The laying nest 50 is fixed in the tubular support 68 on the exterior of the egg laying plate 66, which is itself fixed in the tubular support 68. The odoriferous source 62 is arranged in the tubular support, at the side of the laying nest opposite the egg laying zones 52. The tubular support 68 is then closed with the stopper 69 at the side opposite the cage so as to prevent the diffusion of odours from this side.

The connection aperture 29 is then connected to a source of flies, which is not illustrated, for example, by means of a flexible pipe.

After the connection aperture 29 has been opened, flies M are thus introduced, via the connection aperture 29, into the cage 20. The connection aperture 29 is then closed so as to enclose the flies in the cage. The pipe can be disconnected.

The dimensions of the cage 20 allow the flies to fly and to mate. In particular, when a female fly and a male fly prepare to mate, they fall together onto the base surface 30. The limited height of the lateral wall prevents this fall from being excessively violent.

At least until the eggs are laid, a nutritional liquid, preferably water, produced by the liquid source 70, is introduced into the cage 20. The quantity of liquid introduced is controlled in order to prevent any accumulation of liquid in the bottom of the container 22.

The light source 80 illuminates the interior of the cage through the grill 42 of the cover 24. The tubular support 68 protects the egg laying apertures from any direct illumination by the light source 80.

When they are ready to lay eggs, the female flies search for micro-cavities which are defined by re-entrant edges in order to deposit their eggs there. However, the internal surface of the container 22 is smooth and its orientation changes progressively, including in the junction zones 35 between the faces of the lateral wall and in the junction zones 36 between the lateral wall and the base wall. It does not comprise such re-entrant edges or comprises very small quantities thereof.

Furthermore, the odoriferous source 62 emits an odour which passes through the laying nest 50 via the channels 64, then passes through the lateral wall, in this instance the egg laying plate 66, in order to be introduced into the cage 20 via the egg laying apertures 54. This odour attracts the female flies.

Furthermore, the female flies search for the micro-cavities which are least exposed to light and are therefore attracted by the shaded zone produced by the tubular support 68 when the light source 80 is above the cage 20. The female flies on the point of laying eggs are therefore attracted towards the egg laying apertures both by the attractive odour and by the shade.

When a fly M reaches a location near an egg laying aperture 54, it can observe, through the egg laying aperture 54, the presence of an egg laying zone 52 which has a micro-cavity, in the shade, and from which the attractive odour appears to come. Therefore, it is powerfully encouraged to deposit its eggs at that location.

To this end, as illustrated in Figure 4, it introduces its ovipositor in the egg laying aperture 54. The egg laying aperture 54 is sufficiently narrow to prevent the fly from leaving the cage. However, it allows it to reach the egg laying zone 52 in order to deposit its eggs O at that location.

Subsequently, the fly returns to the cage 20.

At any time, an operator can access the laying nest 50 without having to enter the cage 20, and without any risk that a fly can leave the cage. The operator therefore retrieves the eggs without disturbing the life of the flies inside the cage, for example, by removing the laying nest of the tubular support and then by shaking it. He/she can then place the eggs in incubation in order to ensure the hatching of the larvae, then their rearing. Finally, he/she can process the adult larvae in order to obtain the desired products.

As now clearly appears, the invention provides an egg laying device which is highly suitable for a method of entomoculture using flies.

The eggs can be retrieved at any time in a simple manner without disturbing the flies in the cage. The form of the cage, and in particular the limitation of the length of the re-entrant edges, limits the undesirable clutches of eggs and therefore makes it easier to maintain the egg laying device.

Tests have shown that the flies from the same batch mate and lay eggs substantially simultaneously, which improves the productivity.

Naturally, the invention is not limited to the embodiments which are described and illustrated and which are provided only for illustrative purposes. In particular, the BSF fly is the fly which is preferentially involved in the invention, but the invention is not limited to this fly.

The form and number of laying nests, the form of the support used to fixedly join the laying nest to the cage, the number and form of the egg laying apertures are not limiting.

## Claims

1. Egg laying device for flies, the device comprising:
- a fly cage (20) comprising a container (22) comprising:
- a base wall (26) which defines a base surface (30) which is orientated towards the interior of the cage (20), and
- a lateral wall (28) which surrounds the base surface (30) and which defines a lateral surface (33) which is orientated towards the interior of the cage, and
- a laying nest (50) which is fixed to the cage and which is intended to receive eggs laid by flies (M) which are enclosed in the cage (20),
in which device the base surface and/or the lateral surface has/have, over more than 90% of the area(s) thereof, a radius of curvature greater than 0.5 cm,
wherein the base surface (30) extends in a plane in an uninterrupted manner as far as a junction zone (36) defined between the base surface (30) and the lateral surface (33),
wherein the base surface (30) does not have any raised portions or depressions, the height of which measured perpendicularly to the general plane of the base surface being greater than 10 mm,
**characterized in that**
the junction zone (36) defined between the lateral surface (33) and the base surface (30), has at any point a radius of curvature greater than 1 cm and **in that** the lateral wall (28) comprises a plurality of planar faces, a junction zone (35) between two adjacent faces having at any point a radius of curvature greater than 1 cm.

2. Device according to the preceding claim, wherein an internal surface of the container (30, 33, 35, 36), constituted by the base surface (30), the lateral surface (33) and the junction zones (35, 36), has one or more re-entrant edges, the cumulative length *La* of the one or more re-entrant edges being such that the ratio of the cumulative length *La* to the area S of the internal surface of the container, respectively, *La*/*S,* is less than 1,200 mm/m².

3. Device according to either of the preceding claims, wherein the lateral surface has, over more than 99% of the area thereof, a radius of curvature greater than 2 cm.

4. Device according to the immediately preceding claim, wherein the lateral surface has, over more than 99% of the area thereof, a radius of curvature greater than 5 cm.

5. Device according to claim 2, wherein the height (h₃₃) of the internal surface (30, 33, 35, 36) of the container is between 0.4 m and 1.8 m.

6. Device according to any one of the preceding claims, wherein a cover (24) for closing the container (22) is fixed to the lateral wall by means of a hook-and-loop strip (40₂₂, 40₂₄).

7. Device according to any one of the preceding claims, wherein the base surface and/or the lateral surface has/have a white colour or a colour in the colour spectrum between 472 nm and 600 nm.

8. Installation comprising a unit (12) for producing flies, a unit (14) for producing eggs which are laid by the flies, a unit (16) for producing young larvae from the eggs, a unit (18) for producing adult larvae from the young larvae, and a unit (19) for processing the adult larvae in order to produce a protein-containing product and/or a fertiliser and/or an oil, the unit for producing eggs comprising an egg laying device according to any one of the preceding claims.

9. Installation according to the immediately preceding claim, wherein the flies are *Hermetia illucens* flies.

## Patentansprüche

1. Eierlegevorrichtung für Fliegen, wobei die Vorrichtung Folgendes umfasst:
- einen Fliegenkäfig (20), der ein Behältnis (22) umfasst, umfassend:
- eine Basiswand (26), die eine Basisfläche (30) definiert, welche zum Inneren des Käfigs (20) ausgerichtet ist, und
- eine laterale Wand (28), die die Basisfläche (30) umgibt und die eine laterale Fläche (33) definiert, welche zum Inneren des Käfigs ausgerichtet ist, und
- ein Legenest (50), das an dem Käfig fixiert ist und das dazu vorgesehen ist, von den in dem Käfig (20) eingeschlossenen Fliegen (M) gelegte Eier aufzunehmen, wobei in der Vorrichtung die Basisfläche und/oder die laterale Fläche über mehr als 90% ihrer Fläche(n) einen Krümmungsradius von über 0,5 cm aufweist/aufweisen,
wobei sich die Basisfläche (30) ununterbrochen in einer Ebene bis zu einer zwischen der Basisfläche (30) und der lateralen Fläche (33) definierten Verbindungszone (36) erstreckt,
wobei die Basisfläche (30) keine erhabenen Teile oder Vertiefungen aufweist, deren Höhe senkrecht zu der allgemeinen Ebene der Basisfläche gemessen größer als 10 mm ist,
**dadurch gekennzeichnet,**
**dass** die zwischen der lateralen Fläche (33) und der Basisfläche (30) definierte Verbindungszone (36) an beliebiger Stelle einen Krümmungsradius von über 1 cm aufweist, und
**dass** die laterale Wand (28) mehrere planare Flächen umfasst, wobei eine Verbindungszone (35) zwischen zwei benachbarten Flächen an beliebiger Stelle einen Krümmungsradius von über 1 cm aufweist.

2. Vorrichtung nach dem vorhergehenden Anspruch, wobei eine Innenfläche des durch die Basisfläche (30), die laterale Fläche (33) und die Verbindungszonen (35, 36) gebildeten Behältnisses (30, 33, 35, 36) einen oder mehrere einspringende Ränder aufweist, wobei die kumulative Länge La des einen oder der mehreren einspringenden Ränder derart ist, dass das Verhältnis der kumulativen Länge La zu der Fläche S der Innenfläche des Behältnisses, bzw. La/S, kleiner ist als 1.200 mm/m².

3. Vorrichtung nach einem der vorhergehenden Ansprüche, wobei die laterale Fläche über mehr als 99% ihrer Fläche einen Krümmungsradius von über 2 cm aufweist.

4. Vorrichtung nach dem unmittelbar vorhergehenden Anspruch, wobei die laterale Fläche über mehr als 99% ihrer Fläche einen Krümmungsradius von über 5 cm aufweist.

5. Vorrichtung nach Anspruch 2 wobei die Höhe (h₃₃) der Innenfläche (30, 33, 35, 36) des Behältnisses zwischen 0,4 m und 1,8 m liegt.

6. Vorrichtung nach einem der vorhergehenden Ansprüche, wobei ein Deckel (24) zum Verschließen des Behältnisses (22) mittels eines Klettverschlussbands (40₂₂, 40₂₄) an der lateralen Wand fixiert ist.

7. Vorrichtung nach einem der vorhergehenden Ansprüche, wobei die Basisfläche und/oder die laterale Fläche eine weiße Farbe oder eine Farbe im Farbspektrum zwischen 472 nm und 600 nm aufweist/aufweisen.

8. Anlage, umfassend eine Einheit (12) zum Produzieren von Fliegen, eine Einheit (14) zum Produzieren von Eiern, die von den Fliegen gelegt werden, eine Einheit (16) zum Produzieren von Junglarven aus den Eiern, eine Einheit (18) zum Produzieren von adulten Larven aus den Junglarven und eine Einheit (19) zum Verarbeiten der adulten Larven, um ein proteinhaltiges Produkt und/oder einen Dünger und/oder ein Öl zu produzieren, wobei die Einheit zum Produzieren von Eiern eine Eiablagevorrichtung nach einem der vorhergehenden Ansprüche umfasst.

9. Anlage nach dem unmittelbar vorhergehenden Anspruch, wobei die Fliegen *Hermetia illucens* sind.

## Revendications

1. Dispositif de ponte pour mouches, le dispositif comprenant :
une cage à mouches (20) comprenant un contenant (22) comprenant :
une paroi de base (26) qui définit une surface de base (30) qui est orientée vers l'intérieur de la cage (20), et
une paroi latérale (28) qui entoure la surface de base (30) et qui définit une surface latérale (33) qui est orientée vers l'intérieur de la cage, et
un nid de ponte (50) qui est fixé à la cage et qui est destiné à recevoir des œufs pondus par les mouches (M) qui sont enfermées dans la cage (20),
dans lequel dispositif la surface de base et/ou la surface latérale ont, sur plus de 90 % de leur superficie, un rayon de courbure supérieur à 0,5 cm,
la surface de base (30) s'étendant dans un plan de manière ininterrompue jusqu'à une zone de jonction (36) définie entre la surface de base (30) et la surface latérale (33), la surface de base (30) ne présentant pas de parties surélevées ou de dépressions dont la hauteur mesurée perpendiculairement au plan général de la surface de base est supérieure à 10 mm,
**caractérisé en ce que**
la zone de jonction (36) définie entre la surface latérale (33) et la surface de base (30) présente en tout point un rayon de courbure supérieur à 1 cm et **en ce que** la paroi latérale (28) comprend une pluralité de faces planes, une zone de jonction (35) entre deux faces adjacentes présentant en tout point un rayon de courbure supérieur à 1 cm.

2. Dispositif selon la revendication précédente, une surface interne du contenant (30, 33, 35, 36), constituée par la surface de base (30), la surface latérale (33) et les zones de jonction (35, 36), présentant un ou plusieurs bords rentrants, la longueur cumulée *La* du ou des bords rentrants étant telle que le rapport de la longueur cumulée *La* à la *superficie S* de la surface interne du contenant, respectivement, *La*/*S,* est inférieur à 1 200 mm/m².

3. Dispositif selon l'une des revendications précédentes, la surface latérale présentant, sur plus de 99 % de sa superficie, un rayon de courbure supérieur à 2 cm.

4. Dispositif selon la revendication immédiatement précédente, la surface latérale présentant, sur plus de 99 % de sa superficie, un rayon de courbure supérieur à 5 cm.

5. Dispositif selon la revendication 2, la hauteur (h₃₃) de la surface interne (30, 33, 35, 36) du contenant étant comprise entre 0,4 m et 1,8 m.

6. Dispositif selon l'une quelconque des revendications précédentes, un couvercle (24) destiné à fermer le contenant (22) étant fixé à la paroi latérale au moyen d'une bande auto-agrippante (40₂₂, 40₂₄).

7. Dispositif selon l'une quelconque des revendications précédentes, la surface de base et/ou la surface latérale ayant une couleur blanche ou une couleur dans le spectre des couleurs entre 472 nm et 600 nm.

8. Installation comprenant une unité (12) de production de mouches, une unité (14) de production d'oeufs qui sont pondus par les mouches, une unité (16) de production de jeunes larves à partir des œufs, une unité (18) de production de larves adultes à partir des jeunes larves, et une unité (19) de traitement des larves adultes en vue de produire un produit contenant des protéines et/ou un engrais et/ou une huile, l'unité de production d'œufs comprenant un dispositif de ponte selon l'une quelconque des revendications précédentes.

9. Installation selon la revendication immédiatement précédente, les mouches étant des mouches *Hermetia illucens.*
